# EUROPEAN PATENT SPECIFICATION

(11) **EP 4 029 996 B1**
(45) Date of publication and mention of the grant of the patent: **16.07.2025**
(21) Application number: 21215059.3
(22) Date of filing: 16.12.2021
(51) Int. Cl.: E02D 3/02, E02D 3/026

(54) **METHOD FOR COMPACTING SOIL AND SOIL COMPACTOR**
VERFAHREN ZUM VERDICHTEN VON BODEN UND BODENVERDICHTUNGSVORRICHTUNG
PROCÉDÉ DE COMPACTAGE DE SOL ET COMPACTEUR DE SOL

(30) Priority: 29.12.2020 FI 20206380
(43) Date of publication of application: 20.07.2022
(73) Proprietor: Novatron Oy, 33960 Pirkkala (FI)
(72) Inventor: Moisio, Petri, 33960 Pirkkala (FI); Vesanen, Mikko, 33960 Pirkkala (FI); Kolu, Antti, 33960 Pirkkala (FI)
(74) Representative: Kolster Oy Ab

(56) References cited:
- WO-A1-95/16227
- JP-A- 2020 176 401
- US-A- 5 646 844
- US-A1- 2020 217 033
- US-A1- 2020 356 088

## Description

### FIELD OF THE INVENTION

The invention relates to a method for compacting soil by a soil compactor comprising at least one soil compacting device.

The invention relates also to a soil compactor.

### BACKGROUND OF THE INVENTION

Soil compactors may be used for compacting soil for example in earthworks relating to earth fillings, such as in connection with earthworks relating to a construction of a foundation for a building, a landscaping, a landfill construction or a road construction. The soil compacting is used for ensuring a sufficient compaction level of soil, i.e. a sufficient degree of compaction of soil, that provides a sufficient compactness, endurance or bearing or load capacity of soil in view of an intended purpose of use of the finished earthwork.

Depending on the earthwork or a part of the earthwork to be carried out there are a variety of methods for achieving the sufficient compaction level of soil. According to an embodiment the soil compacting may comprise driving the soil compactor a number of times over each part of an area to be compacted, whereby driving the soil compactor a sufficient number of times over each part of the area to be compacted a weight of the soil compactor provides the sufficient compaction level of soil. This embodiment may be applicable for example when a thickness of a deposited layer of loose soil to be compacted is small or an effect of the compacting is intended to extend only to a surface layer of the area to be compacted. According to another embodiment the soil compacting may comprise driving the soil compactor a number of times over each part of the area to be compacted as well as producing, by at least one soil compacting device forming part of the soil compactor, pressure pulses or vibrations to the soil to be compacted while driving the soil compactor, whereby driving the soil compactor a sufficient number of times over each part of the area to be compacted, the weight of the soil compactor and the pressure pulses or vibrations travelling in the soil together provide the sufficient compaction level of soil. This embodiment may be applicable for example when an effect of the compacting is intended to extend substantially deeper in the soil than only in the layer of soil being substantially visible. For example US-publications US-2020/356088 A1 and US2020/217033 A1 and JP-publication JP-2020176401 A relate to compacting soil at a worksite.

One problem associated with the soil compacting is to ensure that the sufficient compaction level is achieved. When the achieved compaction level is dependent on driving the soil compactor the sufficient number of times over each part of the area to be compacted, it should be verified that the soil compactor is actually driven that sufficient number of times over each part of the area to be compacted and that there are no parts of the area to be compacted that are not driven over by the soil compactor the sufficient number of times. If the achieved compaction level is also dependent on the pressure pulses or vibrations produced to the soil to be compacted, it should also be verified that the intended compaction level, i.e. a target compaction level, of the soil S has been provided. On the other hand, at the same time it should also be verified that no excessive compaction effect is directed to the soil to be compacted, because instead of increasing the compaction level of the soil the excessive compaction effect may start to break the structure of the layer of the soil to be compacted.

Therefore, there is a need for a simple solution for controlling the soil compacting such that a sufficient but not excessive compaction level of soil is achieved.

### BRIEF DESCRIPTION OF THE INVENTION

An object of the present invention is to provide a novel method for compacting soil and a novel soil compactor.

The invention is characterized by the features of the independent claims.

The invention is based on the accurate determination of the location and travel data of the at least one soil compacting device in the work order area with a concurrent measurement of the achieved compaction level.

An advantage of the invention is an accurate knowledge about the achieved compaction level at different areas in the work order area for ensuring that a sufficient but not excessive compaction level of soil is achieved.

Some embodiments of the invention are disclosed in the dependent claims.

According to an embodiment of the method for compacting soil by a soil compactor comprising at least one soil compacting device, the method comprises generating an as-built model of compaction level covering at least a work order area to be compacted, determining boundaries for the work order area to be compacted, determining location data for an area over which the at least one soil compacting device travels, generating an up-to-date travel path to be driven to cover the work order area to be compacted by the at least one soil compacting device at least once by using compactor operation for compacting soil and measurement function for measuring the compaction level of soil while compacting, and driving the soil compactor according to the up-to-date travel path, wherein the method further comprises saving to the as-built model of compaction level at least a location-specific as-built compaction level of soil and determined boundaries for the work order area to be compacted.

According to an embodiment of the method, the method comprises generating an as-built model of compaction level covering at least a work order area to be compacted, determining boundaries for the work order area to be compacted, determining location data for an area over which the at least one soil compacting device travels. generating an up-to-date travel path to be driven to cover the work order area to be compacted by the at least one soil compacting device at least once by using compactor operation for compacting soil and measurement function for measuring the compaction level of soil while compacting, driving the soil compactor according to the up-to-date travel path, wherein the method further comprises saving to the as-built model of compaction level at least a location-specific as-built compaction level of soil, updating the as-built model of compaction level substantially continuously by at least the location-specific as-built compaction level of soil, and updating the up-to-date travel path according to the updated as-built model of compaction level.

According to an embodiment of the method, the up-to-date travel path covers the work order area such a number of times that the location-specific as-built compaction level of soil exceeding a threshold compaction level of soil covers the work order area.

According to an embodiment of the method, the as-built model of compaction level further comprises boundaries for the areas exceeding the threshold compaction level of soil.

According to an embodiment of the method, the as-built model of compaction level further comprises at least one of: determined boundaries for the work order area to be compacted or boundaries for the areas exceeding the threshold compaction level of soil.

According to an embodiment of the method, generating of the up-to-date travel path is in addition to the work order area to be compacted based at least on physical properties to control the soil compactor and physical properties of the at least one soil compacting device of the soil compactor.

According to an embodiment of the method, the saved location-specific as-built compaction level of soil is updated to the as-built model of compaction level by a subsequent level of location specific as-built compaction level of soil by the at least one of replacing or rewriting.

According to an embodiment of the method, it is determined the threshold compaction level of soil being exceeded if percentage change in between two subsequent levels of measured location-specific compaction level of soil is below determined threshold percentage.

According to an embodiment of the method, the as-built model of compaction level further comprises at least one of a location-specific as-built height level or a location-specific number of levels of measured location-specific as-built compaction levels of soil.

According to an embodiment of the method, the boundaries for the work order area to be compacted is determined by at least one of manually driven and selecting at least one of rightmost or leftmost edge of the area over which the at least one soil compacting device travels, user defining via a user interface, indication in an earthworks information model or a combination of the previous.

According to an embodiment of the method, the up-to-date travel path contains transitions where at least one of the compactor operation or the measurement function is disabled.

According to an embodiment of the method, the up-to-date travel path comprises driving with compactor operation and without compactor operation.

According to an embodiment of the method, the as-built model of compaction level may be layered on at least one of an earthworks information model or a map.

According to an embodiment of the method, the soil compactor is driven at least one of manually, semiautomatically or automatically.

According to an embodiment of the soil compactor, the soil compactor comprises at least one soil compacting device with measurement arrangement for compacting soil and measuring compaction level of soil while compacting, a navigating arrangement for providing location and direction of travel data of the at least one soil compacting device, driving arrangement for driving the soil compactor, and a control system, wherein the control system comprises at least one processor and at least one memory including computer program code, the at least one memory and the computer program code configured to with the at least one processor, cause the control system at least to generate an as-built model of compaction level covering at least a work order area to be compacted, receive boundaries for the work order area to be compacted, generate an up-to-date travel path to be driven to cover the work order area to be compacted at least once, and save to the as-built model of compaction level at least a location-specific as-built compaction level of soil and determined boundaries for the work order area to be compacted.

According to an embodiment of the soil compactor, the soil compactor comprises at least one soil compacting device with measurement arrangement for compacting soil and measuring compaction level of soil while compacting, a navigating arrangement for providing location and direction of travel data of the at least one soil compacting device, driving arrangement for driving the soil compactor and a control system, wherein the control system comprises at least one processor and at least one memory including computer program code, the at least one memory and the computer program code configured to with the at least one processor, cause the control system at least to generate an as-built model of compaction level covering at least a work order area to be compacted, receive boundaries for the work order area to be compacted; generate an up-to-date travel path to be driven to cover the work order area to be compacted at least once, and save to the as-built model of compaction level at least a location-specific as-built compaction level of soil, update the as-built model of compaction level substantially continuously by at least the location-specific as-built compaction level of soil, and update the up-to-date travel path according to the updated as-built model of compaction level.

According to an embodiment of the soil compactor, the control system is further configured to receive an earthworks information model covering at least the work order area indicating the area to be compacted.

According to an embodiment of the soil compactor, the soil compactor is driven at least one of: manually, semiautomatically or automatically.

### BRIEF DESCRIPTION OF THE DRAWINGS

In the following the invention will be described in greater detail by means of preferred embodiments with reference to the accompanying drawings, in which
Figure 1 shows schematically a side view of a soil compactor;
Figure 2 shows schematically an upper view of a worksite and a soil compactor therein;
Figure 3 shows schematically a system level illustration of a soil compactor and devices or applications relating thereto for compacting soil;
Figure 4 shows schematically an embodiment of a method for compacting soil; and
Figure 5 shows schematically another embodiment of a method for compacting soil.

For the sake of clarity, the figures show some embodiments of the invention in a simplified manner. Like reference numerals identify like elements in the Figures.

### DETAILED DESCRIPTION OF THE INVENTION

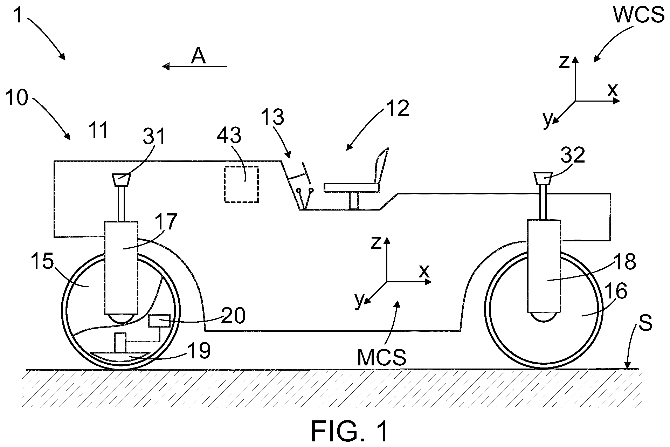

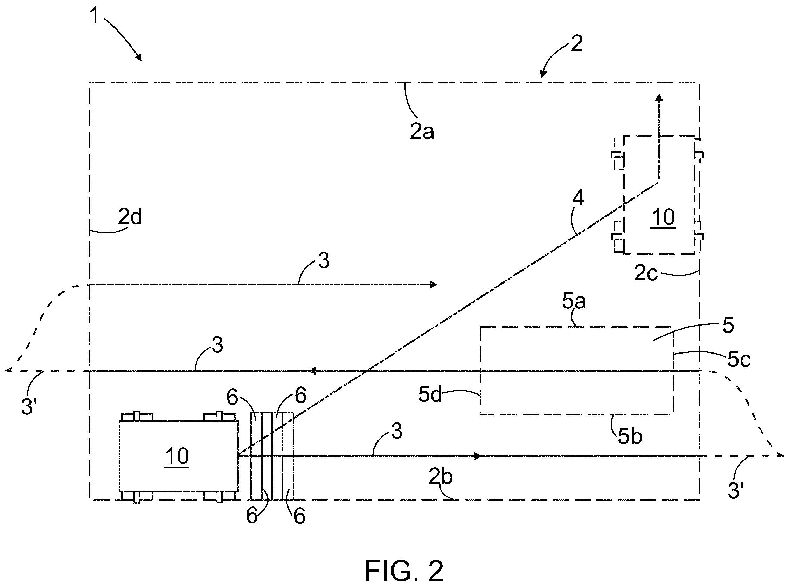

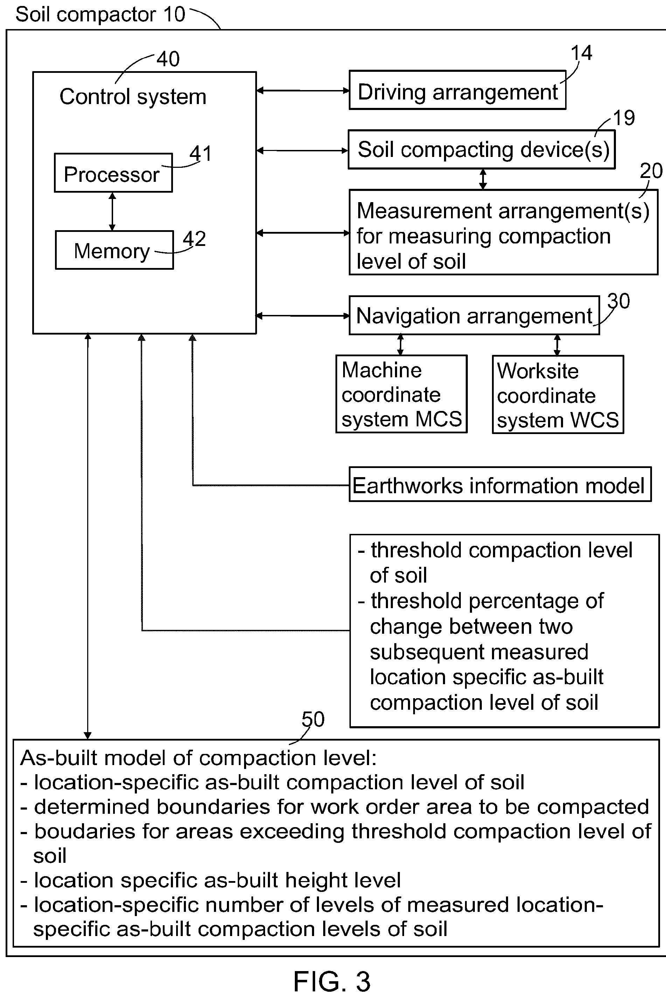

Figure 1 is a schematic side view of a soil compactor 10 at a worksite 1 wherein the soil compactor 10 is intended to be operated. The worksite 1 comprises at least one area at which the soil compacting is about to take place, that area being herein referred to a work order area 2. The work order area 2 thus forms at least a part of an area of the worksite 1. Figure 2 shows schematically an upper view of the worksite 1 and the work order area 2 wherein the soil compactor 10 is about to operate. In Figure 2 the work order area 2 is shown very schematically by a box drawn with a broken line and denoted with reference sign 2. The horizontal 2a, 2b and vertical 2c, 2d lines of the work order area 2 denote boundaries of the work order area 2. Figure 3 shows schematically a system level illustration of a soil compactor 10 and devices or applications relating thereto for compacting soil.

The soil compactors may be used for compacting soil for example in earthworks relating to earth fillings, such as in connection with earthworks relating to a construction of a foundation for a building, a landscaping, a landfill construction or a road construction, or in other kind of earthworks wherein compacting soil is applied. The soil compacting is used for ensuring a sufficient compaction level of soil, i.e. a sufficient degree of compaction of soil, that provides a sufficient compactness, endurance or bearing or load capacity of soil in view of an intended purpose of use of the finished earthwork.

The feature or term compacting soil may herein refer to a compacting of a structural layer, such as a lowest structural layer of an excavation. The feature or term compacting soil may herein also refer to a compacting of one or more layers of other material(s) deposited on the ground or on top of each other. Therefore, according to an embodiment the compacting soil may be subjected to the ground, at some level thereof, whereby the intended compaction of soil may be extended only very close to a visible surface of the ground, such as very close to a surface of the lowest structural layer of the excavation, or alternatively more or less deeper in the ground. Alternatively, according to an embodiment the compacting of soil may be subjected to one or more layers of material(s) deposited on top of the ground or on top of other layer(s) of material(s) already deposited on top of the ground, whereby the intended compaction of soil may be extended only up to a depth of the topmost layer(s) of deposited material(s), or alternatively more or less deeper also into lower layers of deposited material(s) and possibly even up to the ground below all the deposited material layers. Alternatively, according to an embodiment the compacting of soil may be subjected to the topmost layer(s) of material(s) deposited and forming the finished surface of the earthwork, whereby the intended compaction of soil may be extended only up to the topmost layer(s) of deposited material(s), or alternatively more or less deeper also into lower layers of deposited material(s) and possibly even up to the ground below all the deposited material layers. Therefore, according to an embodiment, the compacting soil may also refer to compacting of deposited asphalt, bitumen or the like. The term "soil" may thus refer to a solid or a substantially solid ground or layers of deposited loose materials, such as crushed stone, gravel, asphalt, bitumen, mould, stone ash or the like.

Referring particularly to Figures 1 and 3, the soil compactor 10 comprises a body or a frame 11 that inter-operatively connects and associates various physical or structural features that enable the soil compactor 10 to function. These features include for example an engine, such as a combustion engine or an electric motor or a hybrid combination thereof as well as a power transmission system to generate and transmit the necessary power to move the soil compactor 10 at the worksite 1, for example on the soil S to be compacted. For the sake of clarity, the power generation and transmission system, those being as such generally known by a person skilled in the art, are not disclosed in Figures.

The soil compactor 10 may also comprise a control cabin 12 for a user or an operator of the soil compactor 10, the control cabin 12 encompassing driving means 13 enabling the user to drive the soil compactor 10. These driving means 13 may include for example a steering wheel, a shift handle, a throttle pedal, a brake pedal, a clutch pedal or other applicable means enabling the driving of the soil compactor 10. In the embodiment of Figure 1 the control cabin 12 is open but it could also be closed or covered.

The soil compactor 10 shown schematically in Figure 1 and described above is a manually operated soil compactor 10 wherein the user of the soil compactor 10 drives the soil compactor 10 substantially continuously and actively for example by applying steering, acceleration and/or deceleration actions. Alternatively, the soil compactor may be a semiautomatically operated soil compactor which at least periodically can operate autonomously without a constant active control actions taken by the user. Furthermore, according to an embodiment, the soil compactor 10 may be an automatically operated soil compactor which can at least primarily operate autonomously but which may also be able to, for example in exceptional circumstances, request from the user for assistance, for example through a control system of the soil compactor 10. In the latter two cases the user may also be able to control the operation of the soil compactor only in a remotely controlled manner, by using for example a dedicated hand-held remote control device, virtual vision glasses and/or data-processing equipment, such as a control computer located in a centralized control centre, that being for example a site office of the worksite 1. The driving means 13, the dedicated hand-held control device, the virtual vision glasses, the control computer and/or any other applicable means enabling the driving of the soil compactor 10 provide or form a driving arrangement 14 for driving the soil compactor 10.

To enable the movement of the soil compactor 10, the illustrated soil compactor 10 comprises a substantially cylindrical first roller drum 15 and a substantially cylindrical second roller drum 16 that are in rolling contact with the soil S, the first roller drum 15 being shown in Figure 1 as partly cut open. Assuming a principal direction of travel of the soil compactor 10 being to the left in Figure 1, as shown schematically with an arrow denoted by reference sign A, the first roller drum 15 provides thereby a front roller drum 15 and the second roller drum 16 provides a rear roller drum 16. The front 15 and the rear 16 roller drums are suspended to the frame 11 of the soil compactor 10 by a front suspension 17 and a rear suspension 18 such that the roller drums 15, 16 are generally transverse or perpendicular to the direction of travel A of the soil compactor 10. At least one of the front roller drum 15 and the rear roller drum 16 is steerable for steering the direction of travel of the soil compactor 10, and at least one of the front roller drum 15 and the rear roller drum 16 forms a drive drum causing the travel or movement of the soil compactor 10 in response to the power transmitted to the respective roller drum for moving the soil compactor 10.

Figure 1 and the description above illustrates only one possible embodiment of the soil compactor and the arrangement of the roller drums in the soil compactor. Any other kind of soil compactor, such as a soil compactor with a centre pivot steering, or any other kind of arrangement of the roller drums, as well as the number of the roller drums is possible in view of the solution for compacting soil disclosed herein.

The soil compactor 10 further comprises a soil compacting device 19 intended to create pressure pulses or vibrations to soil S to be compacted while the soil compactor 10 is moving and the soil compacting device 19 is enabled, i.e. in operation. In the embodiment of Figure 1 the soil compactor 10 comprises a single very schematically illustrated soil compacting device 19 accommodated in the front roller drum 15. The soil compacting device 19 preferably extends substantially over the complete width of the front roller drum 15. There could, however, be an alternative or additional soil compacting device accommodated in the rear 16 roller drum or even being suspended to the frame 11. For generating the pressure pulses or vibrations the soil compacting device 19 may for example comprise a mechanical vibratory system arranged to rotate a number of eccentric masses to generate the vibratory force and thereby cause the pressure pulses or vibrations toward soil S. Alternatively, the vibratory force and thereby the pressure pulses or vibrations toward soil S may for example be generated by an electrically-driven vibratory system. Various soil compacting devices are generally known by a person skilled in the art and therefore they are not discussed herein in more detail.

The soil compactor 10 further comprises a measurement arrangement 20 for measuring compaction level of soil S. The measurement arrangement 20 comprises necessary devices for measuring or determining parameters or variables relating to the determination of the compaction level of soil S. The measurement arrangement 20 is typically arranged in connection with the soil compacting device 19 and/or in connection with the roller drum accommodating the soil compacting device 19. There are various applications available for determining the compaction level of soil S. Some of the applications are based on frequency analysis of vertical drum acceleration amplitudes at fundamental (operating) vibration frequency and its harmonic(s) and possible subharmonics. Some other applications are based on the analysis of vertical drum displacement and drum-soil contact force. In connection with the solution for compacting soil disclosed herein, any application for determining the compaction level of soil S may be applied. Because these applications are either generally known or can easily be found out and applied by a person skilled in the art, they are not discussed herein in more detail.

The soil compactor 10 may further comprise control means or devices for controlling or adjusting an operation of the vibratory system of the soil compacting device in order to control or adjust compaction parameters, such as a power of the pressure pulses or vibrations, a frequency of the operation of the vibratory system, an amplitude of the pressure pulses or vibrations produced or an angle at which the pressure pulses or vibrations are directed towards the soil S, the amplitude of the pressure pulses or vibrations being at least partly dependent on the applied power of the pressure pulses or vibrations. These control means or devices may be implemented for example as part of the driving arrangement 14 of the soil compactor 10 or as part of the soil compacting device 19.

The soil compactor 10 further comprises a navigation arrangement 30 for providing location and direction of travel data of the soil compactor 10, and especially for providing location and direction of travel data of the at least one soil compacting device 19. In addition to the actual location and direction of travel data of the soil compactor 10 and/or of the at least one soil compacting device 19, also data describing inclination of the soil compactor 10 and/or of the at least one soil compacting device 19 during the travel thereof may be provided by the navigation arrangement 30. Referring to the embodiment of Figure 1 the navigation arrangement 30 may comprise a first antenna 31 at the front suspension 17 of the front roller drum 15 and a second antenna 32 at the rear suspension 18 of the rear roller drum 16. Similar antennas may be at similar positions at the opposite side of the soil compactor 10 too. The antennas 31, 32 may form a kind of satellite receiving devices if the soil compactor 10 is intended to be able to utilize a kind of a satellite-based positioning system such as GNSS (Global Navigation Satellite Systems) for the determination of the location and travel data of the soil compactor 10 and especially the location and travel data of the at least one soil compacting device 19 at the worksite 1 or at the work order area 2. In addition to the antennas, or alternatively to the antennas, the navigation arrangement 30 may comprise a number of optical positioning devices, such as at least one camera, stereocamera, lidar, radar and/or tachymeter for the determination of the location and travel data of the soil compactor 10 and especially of the at least one soil compacting device 19 at the worksite 1 or the work order area 2. The navigation arrangement 30 comprising at least one optical positioning device is very useful especially in worksites not allowing reliable operation for satellite-based navigation systems.

For the determination of the location and travel data of the at least one soil compacting device 19 the navigation arrangement 30 may comprise or may be connected with a worksite coordinate system WCS wherein the location and travel data of the soil compactor 10 or the location and travel data of at least the at least one soil compacting device 19 is to be determined in the worksite 1 and specifically in the work order area 2. The navigation arrangement 30 may further comprise or may be connected with a machine coordinate system MCS that may determine the location of at least the at least one soil compacting device 19 relative to the at least one antenna 31, 32 and/or relative to the at least one optical positioning device applied in the soil compactor 10. The machine coordinate system MCS may be fixed to a specific point in the soil compactor 10, for example to a fixing point of the at least one antenna 31, 32 or the at least one optical positioning device such that the positioning provided by the navigation arrangement 30 allows the machine coordinate system MCS to be identified with respect to the worksite coordinate system WCS. The worksite coordinate system WCS and the machine coordinate system MCS are shown schematically in Figure 1. The machine coordinate system MCS may be utilized to provide exact location and travel data of the at least one soil compacting device 19 in case of the location and travel data of the at least one soil compacting device 19 not being considered accurate enough when determined only based on locating the soil compactor 10 in the worksite coordinate system WCS.

The soil compactor 10 further comprises a control system 40. The control system 40 comprises at least one processor 41 and at least one memory 42 including computer program code. The at least one memory 42 and the computer program code is configured to, with the at least one processor, to control the operations of the soil compactor 10 for compacting soil S. The control system 40 may therefore be configured to control the driving or travel of the soil compactor 10 in response to control actions provided by the user of the soil compactor 10 either by the driving means 13 in the control cabin 12 or remotely, including also semiautomatically or automatically provided control actions. Similarly, the control system 40 may be configured to control the operation of the soil compacting device(s) 19, the operation of the measurement arrangement(s) 20 for measuring compaction level of soil S and the operation of the navigation arrangement 30, as well as the operations or functions discussed in more detail below. The control system 40, comprising the at least one processor 41 and the at least one memory 42 may be implemented in at least one control unit 43 assembled in the soil compactor 10 and comprising necessary input/output means for allowing the at least one processor 41 and the at least one memory 42 to communicate with devices or applications connected thereto.

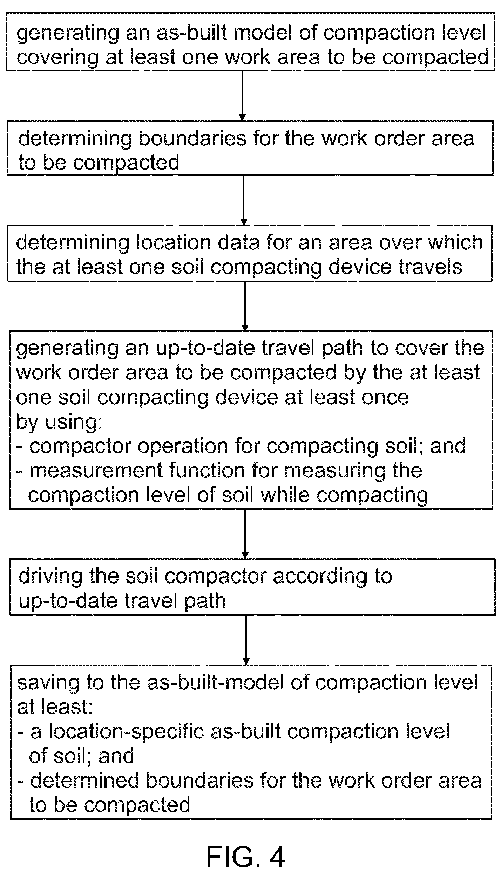

In the following, the solution for compacting soil disclosed herein is considered in more detail, referring especially to Figures 2, 3 and 4, wherein Figure 4 shows schematically an embodiment of a method for compacting soil by a soil compactor comprising at least one soil compacting device.

According to an embodiment, the method comprises generating an as-built model of compaction level covering at least a work order area to be compacted, determining boundaries for the work order area to be compacted, determining location data for an area over which the at least one soil compacting device travels, generating an up-to-date travel path to be driven to cover the work order area to be compacted by the at least one soil compacting device at least once by using compactor operation for compacting soil and measurement function for measuring the compaction level of soil while compacting, and driving the soil compactor according to the up-to-date travel path, wherein the method further comprises saving to the as-built model of compaction level at least a location-specific as-built compaction level of soil and determined boundaries for the work order area to be compacted.

Thus, it is generated an as-built model of compaction level 50 covering at least the work order area 2 to be compacted. The as-built model of the compaction level 50 is a data record or data file that will contain information relating to the work order area 2 to be compacted as well as to work phases planned to be carried out for compacting the soil S in the work order area 2 and to work phases already having been carried out for compacting the soil S in the work order area 2, the work phases referring herein especially to the driving of the soil compactor 10 and/or operating the at least one soil compacting device 19 in the work order area 2. Depending on the compaction level of soil S compaction parameters, such as the power, amplitude, frequency and angle of the pressure pulses or vibrations as provided by the soil compacting device 19 may be controlled during the driving of the soil compactor 10, as discussed in more detail later. The as-built model of the compaction level 50 is created by the soil compactor 10, i.e. by the control system 40 of the soil compactor 10 based on the realized operation of the soil compactor 10 and the soil compacting device 19 therein. The as-built model of compaction level 50 may be updated substantially continuously during the carrying out the compacting work.

The data in the as-built model of the compaction level 50 may provide a level of information connectable with a model of the worksite 1, and especially connectable with a model of the work order area 2 to be compacted. The model of the worksite 1 or the work order area 2 to be compacted may in its simplest form being provided by a generic terrain plain or map, or in its most advanced implementations by digital earthworks information models, such as Built Environment Information Model (BEIM), Geospatial Information Model (GIS), Building Information Modelling (BIM), Infra Building Information Model, Civil Information Model (CIM) and SmartCity Platform. The model of the worksite 1 or the work order area 2, especially the earthworks information models, covering at least the work order area 2 and possibly also comprising indications for the boundaries for the work order area 2 to be compacted, is received by the control system 40, for example from the centralized control centre, that being for example the site office of the worksite 1.

For carrying out the compacting work, and for generating and updating the as-built model of compaction level 50, it is determined the boundaries for the work order area 2 to be compacted. The boundaries for the work order area 2, i.e. the horizontal lines 2a, 2b and the vertical lines 2c, 2d, are preferably not crossed at all during compacting soil S in the work order area 2. Possible enterings of the soil compactor 10 to the work order area 2 and exitings of the soil compactor 10 from the work order area 2, as well as possible changes of courses of the travel of the soil compactor 10 taking place outside the work order area 2 are carried out without compacting soil S. Possible ways to determine the boundaries for the work order area 2 are discussed in more detail later.

Furthermore, for generating and updating the as-built model of compaction level 50 it is determined location data for areas 6 of the work order area 2, which areas 6 may also be referred to as sub-areas 6 of the work order area 2. The work order area 2 is thus divided, during the compacting work, into areas 6 each of which provides a part of the work order area 2 and each of which areas 6 is typically subjected to the compacting effect and the measurement of the compaction level at that area 6. Some areas 6 have been schematically shown in Figure 2 with boxes denoted with reference sign 6. The size of the area 6 may vary but in its minimum the size of the area 6 may correspond to the size of the surface of soil S which the at least one roller drum is arranged to touch at a time or at which the at least one soil compacting device 19 is arranged to cause the compacting effect at a time, whereby in the latter option the area 6 may also be considered to reduce, in the direction of the travel of the soil compactor 10 or the at least one soil compacting device 19, even up to the point being only a line segment having a length corresponding to the width of the soil compactor 10 or the at least one soil compacting device 19 but not necessarily having a substantial length in the direction of the travel of the soil compactor 10 or the at least one soil compacting device 19.

The location data of the area 6 of the work order area 2 over which the at least one soil compacting device 19 of the soil compactor 10 travels enables to store the area-specific information about the compacting work having already been carried out at that area 6 of the work order area 2 and to determine the still required compacting work to be carried out at that area 6 of the work order area 2 so as to finish the compacting work. The area-specific information for the area 6 of the work order area 2, describing the compacting work having already been carried out at that area 6 of the work order area 2, may for example comprise data describing the location of the area 6, the direction of travel of the soil compacting device 19 in the area 6, a width of the drum of the soil compactor 10 into which the soil compacting device 19 is accommodated and/or a width of the actual soil compacting device 19 if that differs essentially from the width of the drum of the soil compactor 10, compaction parameters used in the area 6 and/or the compaction level of soil S achieved at the area 6. In the event of the areas 6 reducing as described above, instead of considering them to be box-like areas they may be considered or described as data points or vectors comprising or being attributed to the determined location and the determined direction of the travel of the soil compactor 10 or the at least one soil compacting device 19. These data points or vectors may possibly further comprise or be attributed to one or more additional pieces of information listed above. These data points or vectors may thus be considered to form an array of data items describing the realized compacting work, each data item comprising or being attributed to pieces of information describing the respective location and direction of the travel of the soil compactor 10 or the at least one soil compacting device 19 at that location, and each data item possibly further comprising or being attributed to one or more additional pieces of information listed above.

It is to be noted herein, that in order to achieve an accurate location data information for the areas 6 to be compacted and for the areas 6 having already been compacted, it is the location and direction of travel data of the actual at least one soil compacting device 19 which is to be considered herein, not a general location and direction of travel data of the soil compactor 10. The location data for the areas 6 is provided by the navigation arrangement 30. If the devices or applications forming at least part of the navigation arrangement 30 do not straightforwardly provide the location and direction of travel data of the soil compacting device 19 accurately enough, the worksite coordinate system WCS and/or the machine worksite coordinate MCS and their mutual relationship may be utilized therein, as disclosed above.

For actually carrying out the intended or planned compacting work it is further generated an up-to-date travel path to be driven to cover the work order area 2 to be compacted by the at least one soil compacting device 19 at least once by using compactor operation for compacting soil S and measurement function for measuring the compaction level of soil S while compacting. The up-to-date travel path to be driven determines a track or route along which the soil compactor 10 or especially the at least one soil compacting device 19 will or should travel in order to provide the intended or planned compaction effect to the soil S in the areas 6 of the work order area 2. The up-to-date travel path to be driven thus comprises at least the track or route along which the soil compactor 10 or especially the at least one soil compacting device 19 will or should travel when the compactor operation of the soil compactor 10 is enabled, i.e. when the at least one soil compacting device 19 is in operation, and when a measurement function for measuring the compaction level of soil S is enabled during compacting, i.e. when the measurement arrangement 20 for measuring the compaction level of soil S is also in operation. The up-to-date travel path is generated such that it is ensured that each area 6 in the work order area 2 will be subjected at least once to the compacting effect of the at least one soil compacting device 19. An up-to-date travel path is shown schematically in Figure 2 by the line denoted with reference sign 3, wherein the intended direction of travel of the soil compactor 10, i.e. the intended direction of travel of the at least one soil compacting device 19, is shown schematically with arrowheads. The portions of the up-to-date travel path 3 remaining outside the vertical borders 2c, 2d of the work order area 2 and denoting a reverse of the direction of travel of the soil compactor 10/ the at least one soil compacting device 19 are shown with broken lines denoted with reference sign 3'. During the reversal of the direction of travel the at least one soil compacting device 19 is not typically in operation.

For carrying out the intended or planned compacting work, the soil compactor 10 is driven according to the up-to-date travel path 3 at the work order area 2 such that at least the at least one soil compacting device 19 implements or follows the up-to-date travel path 3. The driving of the soil compactor 10 may be implemented manually, semiautomatically or automatically. If the actual travel path of the soil compactor 10 or especially the at least one soil compacting device 19 must, for some reason, deviate from the initially determined travel path, the up-to-date travel path 3 is updated accordingly so as to include information describing or determining the travel path along which the soil compactor 10 or especially the at least one soil compacting device 19 has already travelled as well as to include information describing or determining the travel path along which the soil compactor 10 or especially the at least one soil compacting device 19 should yet travel at least once so that the intended or planned compacting work will be finished. The up-to-date travel path 3 is thus able to change during carrying out the compacting work, if necessary.

For completing the compacting work in question, it is saved or stored into the as-built model of compaction level 50 at least the location-specific compaction level of soil S and determined boundaries 2a, 2b, 2c, 2d for the work order area 2 to be compacted. The location-specific compaction level of soil S is a data record or a data file that attributes a specific achieved compaction level to a specific area 6 in the work order area 2, the achieved compaction level being provided by the measurement arrangement 20 for measuring the compaction level of soil S and the location data of the specific area 6, where the achieved compaction level in question relates to is provided by the navigation arrangement 30. The data relating to the location-specific compaction level of soil S is thus a set of information regarding to a particular achieved compaction level at a particular area in the work order area 2 and forms part of the information content of the as-built model of compaction level 50.

The location-specific compaction level of soil S and the determined boundaries 2a, 2b, 2c, 2d for the work order area 2 to be compacted are preferably saved into the as-built model of compaction level 50 substantially continuously during carrying out the compacting work. The data in the as-built model of compaction level 50, such as the data describing the location-specific compaction level of soil S and the boundaries 2a, 2b, 2c, 2d for the work order area 2 to be compacted may be presented as one or more levels of information to be connectable with the model of the worksite 1, especially connectable with the model describing the work order area 2 to be compacted.

Additionally, also the up-to-date travel path 3 of the soil compactor 10 or especially of the soil compacting device 19 may be saved into the as-built model of compaction level 50 at least temporarily during the carrying out the compacting work. In addition, if possible, reasons maybe with pictorial evidence for deviating from the up-to-date travel path may be saved as well into the as-built model of compaction level 50. For example, avoiding detected obstacle with a picture, or any other identification data of the obstacle.

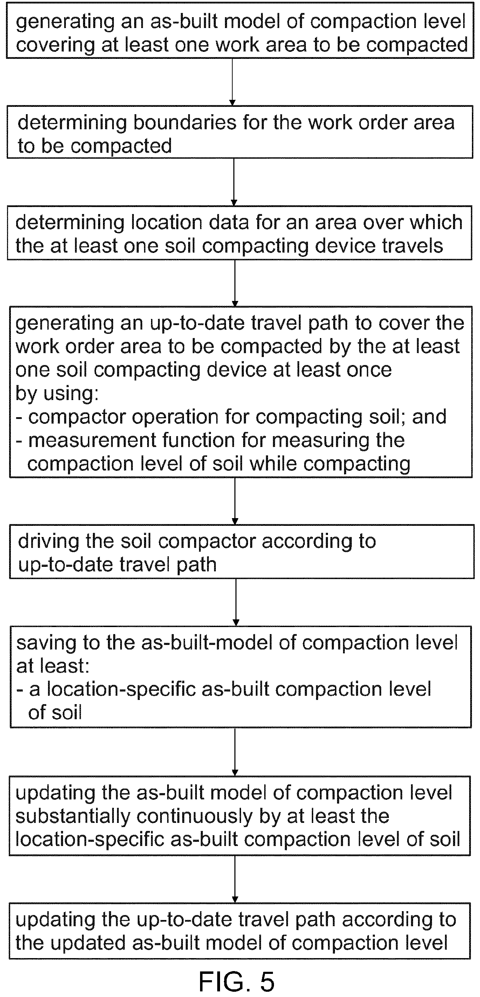

Figure 5 shows schematically another embodiment of the method for compacting soil by a soil compactor comprising at least one soil compacting device. The method according to the embodiment of Figure 5 comprises generating an as-built model of compaction level covering at least a work order area to be compacted, determining boundaries for the work order area to be compacted, determining location data for an area over which the at least one soil compacting device travels, generating an up-to-date travel path to be driven to cover the work order area to be compacted by the at least one soil compacting device at least once by using compactor operation for compacting soil and measurement function for measuring the compaction level of soil while compacting, driving the soil compactor according to the up-to-date travel path, wherein the method further comprises saving to the as-built model of compaction level at least a location-specific as-built compaction level of soil, updating the as-built model of compaction level substantially continuously by at least the location-specific as-built compaction level of soil, and updating the up-to-date travel path according to the updated as-built model of compaction level.

In the embodiment of Figure 5, at least a location-specific compaction level of soil, but not necessarily the determined boundaries for the work order area to be compacted, is saved or stored into the as-built model of compaction level. Because the as-built model of compaction level is updated substantially continuously by at least the location-specific as-built compaction level of soil, and the up-to-date travel path is updated according to the updated as-built model of compaction level and because the soil compactor is driven according to the up-to-date travel path, the information regarding to the boundaries for the work order area to be compacted or already compacted is also expressed or revealed by the information regarding to the location-specific compaction level of soil. Furthermore, the embodiment of Figure 5 explicitly discloses the steps of updating the as-built model of compaction level substantially continuously by at least the location-specific as-built compaction level of soil and updating the up-to-date travel path according to the updated as-built model of compaction level, which steps are comprised in the embodiment of Figure 4 in the step of generating an up-to-date travel path to be driven to cover the work order area to be compacted by the at least one soil compacting device at least once by using compactor operation for compacting soil and measurement function for measuring the compaction level of soil while compacting and in the step of driving the soil compactor according to the up-to-date travel path.

In the solution disclosed the boundaries 2a, 2b, 2c, 2d for the work order area 2 are preferable determined before starting the actual compacting work. i.e. before starting the driving of the soil compactor 10 and the operation of the at least one soil compacting device 19 therein. Alternatively, the actual compacting work and the determination of the boundaries 2a, 2b, 2c, 2d for the work order area 2 may at least partly overlap in time such that some or even all the boundaries 2a, 2b, 2c, 2d for the work order area 2 may be undetermined at a time of starting the compacting work. In that case the compacting work may, for example, be started at a central area of the work order area 2. The compacting work may then proceed towards and up to the boundaries 2a, 2b, 2c, 2d of the work order area 2 after the boundaries 2a, 2b, 2c, 2d of the work order area 2 have been accurately or finally determined. This allows the compacting work at the work order area 2 to be started before the accurate definition of the boundaries 2a, 2b, 2c, 2d of the work order area 2, this allowing to accelerate a progress of the worksite 1.

Generally the solution disclosed provides an accurate knowledge about the achieved compaction level at different areas 6 in the work order area 2 for the whole area of the work order area 2, ensuring that a sufficient but not excessive compaction level of soil S is achieved at the complete work order area 2. At the same time, the soil compactor 10 or at least the at least one soil compacting device 19 may be steered in the work order area 2 very accurately along the up-to-date travel path 3. This means for example that by using the solution presented herein there is no need to have substantially any overlap of the subjected compacting work between neighbouring areas to be compacted in the direction substantially perpendicular to the direction of the travel of the soil compactor 10 or at least the at least one soil compacting device 19 therein, whereas in prior art typically an overlap of at least ten centimetres between the neighbouring areas has been applied to ensure the compacting work being completed at all parts or portions in the area to be compacted.

According to an embodiment, the boundaries for the work order area to be compacted is determined by at least one of: manually driven and selecting at least one of rightmost or leftmost edge of the area over which the at least one soil compacting device travels, user defining via a user interface, indication in an earthworks information model, or a combination of the previous.

The boundaries 2a, 2b, 2c, 2d for the work order area 2 to be compacted may thus be determined by several ways. According to one alternative, the soil compactor 10 is driven manually, either by driving means 13 located in the control cabin 12 or remotely by suitable means, and the at least one boundary 2a, 2b, 2c, 2d of the work order area 2 is indicated and saved into the as-built model of compaction level 50 to be selected at least one of the rightmost or leftmost edge of the area 6 over which the at least one soil compacting device 19 of the soil compactor 10 travels. The at least one soil compacting device 19 may during this kind of boundary determination be either non-operative, i.e. disabled, or in operation, i.e. enabled. In the latter case also the measurement arrangement 20 for measuring the compaction level of soil S would also be activated to measure the provided compaction level for describing the achieved compaction effect. The knowledge of the specific edge, i.e. if it is the question of either the rightmost or leftmost edge or in some situations even both, of the area 6 over which the at least one soil compacting device 19 travels and which edge is thereby intended to determine the specific boundary 2a, 2b, 2c, 2d of the work order area 2, may have a crucial effect on carrying out the compacting work. This is the case for example in a road construction, wherein neighbouring traffic lanes may decline into different directions and it is not allowed to drive the soil compactor 10 over a central line at which the neighbouring traffic lanes meet. The same aspect relates to for example also an edge of a slope or ramp being intended to decline to a specific direction.

According to another alternative a user or operator of the soil compactor 10 defines the boundaries 2a, 2b, 2c, 2d for the work order area 2 via a user interface. The user interface may for example comprise a display and a computer program code configured to draw the boundaries 2a, 2b, 2c, 2d for the work order area 2 in a model of the worksite 1 in response to the definition(s) provided by the user. According to another example, the user interface may comprise a touchscreen at which the user draws the boundaries 2a, 2b, 2c, 2d for the work order area 2 in the model of the worksite 1. The boundaries 2a, 2b, 2c, 2d of the work order area 2 are thereafter saved into the as-built model of compaction level 50.

According to a further alternative the boundaries 2a, 2b, 2c, 2d for the work order area 2 are defined in the earthworks information model and are loaded therefrom to the control system 40 of the soil compactor 10, the boundaries being also saved into the as-built-map of compaction level 50.

The boundaries 2a, 2b, 2c, 2d for the work order area 2 may also be determined by any combination of the alternative ways disclosed above.

Referring again to the up-to-date travel path 3, the up-to-date travel path 3 in the embodiment above comprised at least the track or route along which the soil compactor 10 or especially the at least one soil compacting device 19 will or should travel when the at least one soil compacting device 19 is operated and when the measurement arrangement 20 for measuring the compaction level of soil S is also in operation. However, according to an embodiment, the up-to-date travel path 3 comprises driving with compactor operation enabled or activated and without compactor operation. According to this embodiment there may thus be one or more sections in the travel path which are driven by the soil compactor 10 without the at least one soil compacting device 19 being in operation. These sections of the travel path may for example comprise solid rock, such as bedrock, with no use of trying to compact at all, or sections which have achieved the intended or target compaction level already during the first run or previous runs of the soil compactor 10 in a case of the need for the second or more runs to achieve the intended minimum compaction level for the whole area of the work order area 2. When the at least one soil compacting device 19 is in operation, the compaction parameters of the soil compacting may be controlled or adjusted, as described above, to enhance accomplishment of the intended compaction level of soil S without causing excessive compaction level of soil S.

Furthermore, according to an embodiment, the up-to-date travel path contains transitions where at least one of the compactor operation or the measurement function is disabled, i.e. inactivated. According to this embodiment, the up-to-date travel path 3 may contain sections where the actual driving path of the soil compactor 10 or especially of the at least one soil compacting device 19 deviates, for some reason, from the originally planned driving path. This may take place for example because of an obstacle remaining on the intended up-to-date travel path or because of an area 6 in the work order area 2 that is not yet prepared for the compacting work. In that case the soil compactor 10 may have to skirt the obstacle through another area or move to another area in the work order area 2 by travelling over one or more areas 6 having already been compacted to have the intended compaction level or very close to it, and whereby during this transition movement there is no need to have the at least one soil compacting device 19 and the respective measurement arrangement 20 in operation, unless the weight of the soil compactor 10 as such may cause a significant change in the compaction level of soil S and thereby cause a need to keep the measurement arrangement 20 in operation. Figure 2 shows schematically, with a dot-and-dash line denoted with reference sign 4, a fictitious travel of the soil compactor 10 over the areas remaining on the travel path 3 and considered already at an earlier working phase having been compacted at least once. According to another alternative herein, only the measurement arrangement 20 may be in operation, for example in cases wherein only the weight of the soil compactor 10, without operating the soil compacting device 19, is expected to cause some change in the compaction level of soil S. According to a further alternative herein, only the at least one soil compacting device 19 may be in operation if the effect of the at least one soil compacting device 19 is expected to cause a specific change in the compaction level of soil S, whereby operating the measurement arrangement 20 may be considered unnecessary. The location-specific as-built compaction level of soil S is updated at least at those times when the at least one soil compacting device 19 is in operation.

According to an embodiment, the up-to-date travel path covers the work order area such a number of times that the location-specific as-built compaction level of soil exceeding a threshold compaction level of soil covers the work order area. According to this embodiment the up-to-date travel path 3 is arranged to comprise such a number of runs or travels of the at least one enabled or activated soil compacting device 19 over each area 6 in the work order area 2 that an intended or target compaction level of soil S as determined by the specific predetermined threshold compaction level of soil S is achieved at each area 6 of the work order area 2, i.e. over the complete area of the work order area 2. This means that some areas 6 in the work order area 2 may be travelled over, i.e. subjected to the compaction effect, only once or twice, for example, for achieving the intended or target compaction level of soil S, whereas some other areas 6 in the work order area 2 may have to be subjected to the compaction effect more than two times.

According to an embodiment, the as-built model of compaction level further comprises boundaries for the areas exceeding the threshold compaction level of soil. According to this embodiment, the as-built model of compaction level 50 may further comprise boundaries indicating the areas in the work order area 2 at which the achieved threshold compaction level of soil S exceeds the target or intended compaction level, i.e. the threshold compaction level. Figure 2 discloses schematically an area described with a broken line and denoted with reference sign 5 being located at the up-to-date travel path 3 in the work order area 2 and having boundaries 5a, 5b, 5c, 5d for depicting the area in the work order area 2 wherein the threshold compaction level has been exceeded. By indicating in the as-built model of compaction level 50 the areas in the work order area 2 at which the compaction level exceeds the threshold compaction level, the up-to-date travel path 3 may be updated such that the soil compactor 3 is not controlled to drive over these areas any more, either at all or with the at least soil compacting device 19 being enabled. By this way it may be ensured that no excessive compaction effect is caused at those areas 5 in the work order area 2.

According to an embodiment, the as-built model of compaction level comprises at least one of: determined boundaries for the work order area to be compacted or boundaries for the areas exceeding the threshold compaction level of soil. In other words, according to this embodiment, the as-built model of compaction level comprises the determined boundaries for the work order area to be compacted and/or the boundaries for the areas exceeding the threshold compaction level of soil. The determined boundaries for the work order area to be compacted and the boundaries for the areas exceeding the threshold compaction level of soil have already been discussed in more detail above.

According to an embodiment, the generating of the up-to-date travel path is in addition to the work order area to be compacted based at least on physical properties to control the soil compactor and physical properties of the at least one soil compacting device of the soil compactor. According to this embodiment it may be taken into account, when generating the up-to-date travel path 3, also physical properties to control the soil compactor 3, these physical properties including for example the minimum radius of the turning circle of the soil compactor 10, whereby the up-to-date travel path 3 is not allowed to comprise turns of the soil compactor 10 with a radius smaller than allowed by the minimum radius of the turning circle of the soil compactor 10. Other physical property of the soil compactor 10 that may be taken into account is for example a maximum turning rate and angular acceleration of the drums/centre pivot steering. Furthermore, according to this embodiment, it may be taken into account, when generating the up-to-date travel path 3, also physical properties of the at least one soil compacting device 19 of the soil compactor 10, these including for example an optimum or maximum output force of the soil compacting device 19. The optimum or maximum output force of the soil compacting device 19 may be decisive to determine the number of travels of the soil compacting device 19 over each area 6 in the work order area 2 to achieve the intended or target compaction level of soil S. For example, by using a soil compacting device 19 with a substantially low output force it may be evident already before the starting of the compacting work that at least two travels or runs of the enabled soil compacting device 19 is required to achieve the intended or target compaction level of soil S. Other physical property of the soil compacting device 19 that may be taken into account is for example a range of variation of the frequency, amplitude and/or angle of the pressure pulses or vibrations that the soil compacting device 19 is able to provide.

According to an embodiment, it is updated the saved location-specific as-built compaction level of soil to the as-built model of compaction level by a subsequent level of location specific as-built compaction level of soil by the at least one of: replacing or rewriting. According to this embodiment, the location-specific as-built compaction level of soil S saved previously to the as-built model of compaction level 50 is to be updated during carrying out the compacting work by newer information determined for the location-specific as-built compaction level of soil S.

The newer data describing the achieved compaction level at a specific area 6 of the work order area 2 may replace in the location-specific as-built compaction level of soil S the earlier data describing the previously achieved compaction level at the same specific area 6 of the work order area 2. This alternative may be applicable especially if an angle of the travel of the soil compactor 10 or of at least the travel of the at least one soil compacting device 19 over the specific area 6 of the work order area 2 is the same as during the previous travel over the same area 6. In this case fictitious borders of the area 6 during these two runs may be considered to unite with an acceptable accuracy and the compacting effect and the measurement of the compaction level may be considered to be subjected to the one and same area 6 with the acceptable accuracy.

Alternatively, the newer data describing the achieved compaction level at an area 6 of the work order area 2 may be rewritten in the location-specific as-built compaction level of soil S, in which case also the earlier data describing the previously achieved compaction level at the area 6 of the work order area 2 will be maintained in the updated location-specific as-built compaction level of soil S. This alternative may be applicable if an angle of the travel of the soil compactor 10 or of at least the travel of the at least one soil compacting device 19 over the area 6 of the work order area 2 deviates from the angle of the direction of travel applied during the previous run at the substantially same area 6. However, because of this deviation of the direction of travels between two successive runs the fictitious borders of the area 6 during these two runs does not necessarily unite with an acceptable accuracy and therefore the compacting effect and the measurement of the compaction level may not be considered to be subjected to exactly one and same area 6 with the acceptable accuracy. In this case, a new set of data describing the compacting work subjected to the work order area 2 and to the areas 6 therein may be considered to be useful for storing the information describing the compacting work subjected to the work order area 2 with a desired accuracy.

It is to be noted herein that in both alternatives, in addition to the data describing the compaction level achieved at the specific area 6 also data describing the compaction parameters applied at this area 6 may be saved or stored.

According to an embodiment, it is determined the threshold compaction level of soil being exceeded if percentage change in between two subsequent levels of measured location-specific compaction level of soil is below determined threshold percentage. According to this embodiment, the intended or target compaction level is determined to be achieved if the percentage change between two subsequent measurements for measuring compaction level of soil S is below a predetermined threshold percentage. According to this embodiment a specific achieved compaction level at an area 6 in the work order area 2, such as a compaction level of 140 MPa for example, is not as such under interest but only a percentage change taking place between two successive measurements for measuring compaction level of soil S at the same area 6 in the work order area 2 after the respective two successive travels of the soil compactor 10 over the same area 6 with the at least one soil compacting device 19 having been in operation. If this percentage change between the two subsequent measurements for measuring compaction level of soil S at the same area 6 in the work order area 2 is below the predetermined threshold percentage, it may be considered that the intended or target compaction level has been achieved because no substantial compaction of the specific area 6 of the work order area 2 has not occurred any more.

This embodiment for determining the intended or target compaction level having been achieved based on monitoring the percentage change between the two subsequent measurements for measuring compaction level of soil S at the same area 6 in the work order area 2 may be exemplified with the following table that shows measurements results describing a change in a compaction level of an area 6 to be compacted as measured in a real test run:

| Number of travels over area to be compacted | Change in compaction level of area to be compacted [MPa] | Change in compaction level in per cents |
|---|---|---|
| 1 | 134.1 | 100.0 |
| 2 | 7.9 | 5.9 |
| 3 | 3.7 | 2.8 |
| 4 | 1.1 | 0.8 |
| 5 | 0.9 | 0.7 |
| 6 | 1.1 | 0.8 |

If the set value for the predetermined threshold percentage would have been set to be 8 per cents, the table shows that only after two travels of the soil compacting device over the specific area 6 to be compacted the intended or target compaction level of the area 6 to be compacted would have been achieved, because the change of the compaction level in per cents between the first travel and the second travel is 5.9 per cents, i.e. less than the determined threshold percentage of 8 per cents.

Alternatively, if the set value for the predetermined threshold percentage would have been set to be 3 per cents, the table shows that three travels of the soil compacting device over the area 6 to be compacted would have been needed to achieve the intended or target compaction level of the area 6 to be compacted, because the change of the compaction level in per cents between the second travel and the third travel is 2.8 per cents, i.e. less than the predetermined threshold percentage of 3 per cents.

According to an embodiment, the as-built model of compaction level further comprises at least one of: a location-specific as-built height level or a location-specific number of levels of measured location-specific as-built compaction levels of soil. According to this embodiment, the as-built model of compaction level 50 may comprise a location-specific as-built height level of the area 6 compacted at the work order area 2, i.e. information describing the height level of the area 6 after subjecting the area 6 to the effect of the compacting. The height level of the area 6 may be saved after each travel of the soil compactor 10 or the soil compacting device 19 over the specific area 6 or after the compacting work subjected to that specific area 6 has been completed. Alternatively, or additionally, the as-built model of compaction level 50 may comprise a location-specific number of levels of measured location-specific as-built compaction levels of soil S, i.e. in other words, the number of times the specific area 6 has been subjected to the compacting effect. The information describing the height level and/or the a location-specific number of levels of measured location-specific as-built compaction levels of soil S may also be supplemented with the measured compaction level either in the form of a specific achieved compaction level or in the form of the change of the compaction level between the successive compaction effects subjected to that specific area 6, and/or with information describing the compaction parameters applied at that area 6 and/or with information describing direction of travel of the soil compactor 10 or the at least one soil compacting device 19 over that area 6, possibly including also data describing an inclination angle of the soil compactor 10 or the at least one soil compacting device 19 during the travel thereof over the area 6.

It will be obvious to a person skilled in the art that, as the technology advances, the inventive concept can be implemented in various ways. The invention and its embodiments are not limited to the examples described above but may vary within the scope of the claims.

## Claims

1. A method for compacting soil (S) by a soil compactor (10) comprising at least one soil compacting device (19), the method comprising
generating an as-built model of compaction level (50) covering at least a work order area (2) to be compacted;
determining boundaries (2a, 2b, 2c, 2d) for the work order area (2) to be compacted;
determining location data for an area (6) over which the at least one soil compacting device (19) travels;
generating an up-to-date travel path (3) to be driven to cover the work order area (2) to be compacted by the at least one soil compacting device (19) at least once by using
compactor operation for compacting soil (S) and
measurement function for measuring the compaction level of soil while compacting;
driving the soil compactor (10) according to the up-to-date travel path (3); wherein the method further comprises:
saving to the as-built model of compaction level (50) at least a location-specific as-built compaction level of soil;
updating the as-built model of compaction level (50) substantially continuously by at least the location-specific as-built compaction level of soil; and
updating the up-to-date travel path (3) according to the updated as-built model of compaction level (50).

2. The method according to claim 1, wherein the up-to-date travel path (3) covers the work order area (2) such a number of times that the location-specific as-built compaction level of soil exceeding a threshold compaction level of soil covers the work order area (2).

3. The method according to claim 2, wherein the as-built model of compaction level (50) further comprises at least one of: determined boundaries (2a, 2b, 2c, 2d) for the work order area (2) to be compacted or boundaries for the areas exceeding the threshold compaction level of soil.

4. The method according to any one of claims 1 to 3, wherein generating of the up-to-date travel path (3) is in addition to the work order area (2) to be compacted based at least on:
physical properties to control the soil compactor (10); and
physical properties of the at least one soil compacting device (19) of the soil compactor (10).

5. The method according to any one of claims 1 to 4, wherein it is updated the saved location-specific as-built compaction level of soil to the as-built model of compaction level (50) by a subsequent level of location specific as-built compaction level of soil by the at least one of: replacing or rewriting.

6. The method according to any one of claims 2 to 5, wherein it is determined the threshold compaction level of soil being exceeded if percentage change in between two subsequent levels of measured location-specific compaction level of soil is below determined threshold percentage.

7. The method according to any one of claims 2 to 6, wherein the as-built model of compaction level (50) further comprises at least one of: a location-specific as-built height level or a location-specific number of levels of measured location-specific as-built compaction levels of soil.

8. The method according to any one of claims 1 to 7, wherein the boundaries (2a, 2b, 2c, 2d) for the work order area (2) to be compacted is determined by at least one of:
manually driven and selecting at least one of: rightmost or leftmost edge of the area (6) over which the at least one soil compacting device (19) travels;
user defining via a user interface;
indication in an earthworks information model; or
a combination of the previous.

9. The method according to any one of claims 1 to 8, wherein the up-to-date travel path (3) contains transitions where at least one of: the compactor operation or the measurement function is disabled.

10. The method according to any one of claims 1 to 9, wherein the up-to-date travel path (3) comprises driving with compactor operation and without compactor operation.

11. The method according to any one of claims 1 to 10, wherein the as-built model of compaction level (50) may be layered on at least one of: an earthworks information model or a map.

12. The method according to any one of claims 1 to 11, wherein the soil compactor (10) is driven at least one of: manually, semiautomatically or automatically.

13. A soil compactor (10), the soil compactor (10) comprising:
at least one soil compacting device (19) with measurement arrangement (20) for compacting soil (S) and measuring compaction level of soil while compacting;
a navigating arrangement for providing location and direction of travel data of the at least one soil compacting device (19);
driving arrangement (14) for driving the soil compactor (10); and
a control system (40), wherein the control system (40) comprises at least one processor (41) and at least one memory (42) including computer program code, the at least one memory (42) and the computer program code configured to with the at least one processor (41), cause the control system (40) at least to:
generate an as-built model of compaction level (50) covering at least a work order area (2) to be compacted;
receive boundaries (2a, 2b, 2c, 2d) for the work order area (2) to be compacted;
generate an up-to-date travel path (3) to be driven to cover the work order area (2) to be compacted at least once; and
save to the as-built model of compaction level (50) at least a location-specific as-built compaction level of soil;
update the as-built model of compaction level (50) substantially continuously by at least the location-specific as-built compaction level of soil; and
update the up-to-date travel path (3) according to the updated as-built model of compaction level (50).

14. The soil compactor according to claim 13, wherein the control system (40) is further configured to:
receive an earthworks information model covering at least the work order area (2) indicating the area (6) to be compacted.

15. The soil compactor according to claim 13 or 14, wherein the soil compactor (10) is driven at least one of: manually, semiautomatically or automatically.

## Patentansprüche

1. Verfahren zum Verdichten von Boden (S) durch einen Bodenverdichter (10), der mindestens eine Bodenverdichtungsvorrichtung (19) umfasst, wobei das Verfahren Folgendes umfasst
Erzeugen eines Bestandsmodells eines Verdichtungsmaßes (50), das mindestens einen zu verdichtenden Arbeitsauftragsbereich (2) abdeckt;
Bestimmen von Grenzen (2a, 2b, 2c, 2d) für den zu verdichtenden Arbeitsauftragsbereich (2);
Bestimmen von Standortdaten für einen Bereich (6), über den die mindestens eine Bodenverdichtungsvorrichtung (19) läuft;
Erzeugen eines aktuellen Laufpfades (3), der zu befahren ist, um den von der mindestens einen Bodenverdichtungsvorrichtung (19) zu verdichtenden Arbeitsauftragsbereich (2) unter Verwendung von Folgendem mindestens einmal abzudecken
einer Verdichteroperation zum Verdichten von Boden (S) und
einer Messfunktion zum Messen des Verdichtungsmaßes des Bodens während des Verdichtens;
Fahren des Bodenverdichters (10) gemäß dem aktuellen Laufpfad (3); wobei das Verfahren ferner Folgendes umfasst:
Speichern mindestens eines standortspezifischen Bestandsbodenverdichtungsmaßes im Bestandsmodellverdichtungsmaß (50);
Aktualisieren des Bestandsmodellverdichtungsmaßes (50) im Wesentlichen kontinuierlich durch mindestens das standortspezifische Bestandsbodenverdichtungsmaß, und
Aktualisieren des aktuellen Laufpfades (3) gemäß dem aktualisierten Bestandsmodellverdichtungsmaß (50).

2. Verfahren nach Anspruch 1, wobei der aktuelle Laufpfad (3) den Arbeitsauftragsbereich (2) mit einer derartigen Häufigkeit abdeckt, dass das standortspezifische Bestandsbodenverdichtungsmaß, das einen Bodenverdichtungsmaßschwellwert, den Arbeitsauftragsbereich (2) abdeckt.

3. Verfahren nach Anspruch 2, wobei das Bestandsmodellverdichtungsmaß (50) mindestens eines von Folgendem umfasst: bestimmte Grenzen (2a, 2b, 2c, 2d) für den zu verdichtenden Arbeitsauftragsbereich (2) oder Grenzen für die Bereiche, die den Bodenverdichtungsmaßschwellwert überschreiten.

4. Verfahren nach einem der Ansprüche 1 bis 3, wobei das Erzeugen des aktuellen Laufpfades (3) zusätzlich zu dem zu verdichtenden Arbeitsauftragsbereich (2) mindestens auf Basis von Folgendem zusätzlich erfolgt:
physischen Eigenschaften zum Steuern des Bodenverdichters (10); und
physischen Eigenschaften der mindestens einen Bodenverdichtungsvorrichtung (19) des Bodenverdichters (10).

5. Verfahren nach einem der Ansprüche 1 bis 4, wobei das gespeicherte standortspezifische Bestandsbodenverdichtungsmaß im Bestandsmodellverdichtungsmaß (50) durch mindestens eines vom Ersetzen oder Neuschreiben durch ein nachfolgendes Maß eines standortspezifischen Bestandsbodenverdichtungsmaß aktualisiert wird.

6. Verfahren nach einem der Ansprüche 2 bis 5, wobei bestimmt wird, dass der Bodenverdichtungsmaßschwellwert überschritten ist, wenn eine prozentuale Änderung zwischen zwei aufeinanderfolgenden Maßen eines gemessenen standortspezifischen Bodenverdichtungsmaßes unter einem bestimmten Schwellwertprozentwert liegt.

7. Verfahren nach einem der Ansprüche 2 bis 6, wobei das Bestandsmodellverdichtungsmaß (50) ferner mindestens eines von Folgendem umfasst: einem standortspezifischen Bestandshöhenmaß oder einer standortspezifischen Anzahl von Maßen von gemessenen standortspezifischen Bestandsbodenverdichtungsmaßen.

8. Verfahren nach einem der Ansprüche 1 bis 7, wobei die Grenzen (2a, 2b, 2c, 2d) für den zu vernichtenden Arbeitsauftragsbereich (2) durch mindestens eines von Folgendem bestimmt werden:
manuell Fahren und Auswählen von mindestens einem von Folgendem: einer äußersten rechten oder äußersten linken Kante des Bereichs (6), über die die mindestens eine Bodenverdichtungsvorrichtung (19) läuft;
Benutzerdefinieren via eine Benutzerschnittstelle;
Anzeige in einem Erdarbeiteninformationsmodell; oder
einer Kombination des Vorstehenden.

9. Verfahren nach einem der Ansprüche 1 bis 8, wobei der aktuelle Laufpfad (3) Übergänge enthält, an denen mindestens eines von Folgendem gilt: die Verdichteroperation oder die Messfunktion wird deaktiviert.

10. Verfahren nach einem der Ansprüche 1 bis 9, wobei der aktuelle Laufpfad (3) das Fahren mit Verdichteroperation und ohne Verdichteroperation umfasst.

11. Verfahren nach einem der Ansprüche 1 bis 10, wobei das Bestandsmodellverdichtungsmaß (50) auf mindestens einem von Folgendem geschichtet sein kann: einem Erdarbeiteninformationsmodell oder einer Karte.

12. Verfahren nach einem der Ansprüche 1 bis 11, wobei der Bodenverdichter (10) auf mindestens eine der folgenden Weisen gefahren wird: manuell, halbautomatisch oder automatisch.

13. Bodenverdichter (10), wobei der Bodenverdichter (10) Folgendes umfasst:
mindestens eine Bodenverdichtungsvorrichtung (19) mit einer Messanordnung (20) zum Verdichten von Boden (S) und Messen eines Bodenverdichtungsmaßes während des Verdichtens;
eine Navigationsanordnung zum Bereitstellen eines Standorts und einer Richtung von Fahrdaten der mindestens einen Bodenverdichtungsvorrichtung (19);
eine Fahranordnung (14) zum Fahren des Bodenverdichters (10); und
ein Steuersystem (40), wobei das Steuersystem (40) mindestens einen Prozessor (41) und mindestens einen Speicher (42), der einen Computerprogrammcode beinhaltet, umfasst, wobei der mindestens eine Speicher (42) und der Computerprogrammcode dazu ausgelegt sind, das Steuersystem (40) mit dem mindestens einen Prozessor (41) mindestens zu Folgendem zu veranlassen:
Erzeugen eines Bestandsmodellverdichtungsmaßes (50), das mindestens einen zu verdichtenden Arbeitsauftragsbereich (2) abdeckt;
Empfangen von Grenzen (2a, 2b, 2c, 2d) für den zu verdichtenden Arbeitsauftragsbereich (2);
Erzeugen eines aktuellen Laufpfades (3), der zu befahren ist, um den zu verdichtenden Arbeitsauftragsbereich (2) mindestens einmal abzudecken; und
Speichern mindestens eines standortspezifischen Bestandsbodenverdichtungsmaßes im Bestandsmodellverdichtungsmaß (50);
Aktualisieren des Bestandsmodellverdichtungsmaßes (50) im Wesentlichen kontinuierlich durch mindestens das standortspezifische Bestandsbodenverdichtungsmaß; und
Aktualisieren des aktuellen Laufpfades (3) gemäß dem aktualisierten Bestandsmodellverdichtungsmaß (50).

14. Bodenverdichter nach Anspruch 13, wobei das Steuersystem (40) ferner zu Folgendem ausgelegt ist:
Empfangen eines Erdarbeiteninformationsmodells, das mindestens den Arbeitsauftragsbereich (2) abdeckt, der den zu vernichtenden Bereich (6) anzeigt.

15. Verfahren nach einem der Ansprüche 13 oder 14, wobei der Bodenverdichter (10) mindestens in einer der folgenden Weisen gefahren wird: manuell, halbautomatisch oder automatisch.

## Revendications

1. Procédé de compactage de sol (S) par un compacteur de sol (10) comprenant au moins un appareil de compactage de sol (19), le procédé comprenant les étapes suivantes :
générer un modèle tel que construit d'un niveau de compactage (50) couvrant au moins une zone d'ordre de travail (2) à compacter ;
déterminer des limites (2a, 2b, 2c, 2d) de la zone d'ordre de travail (2) à compacter ;
déterminer des données d'emplacement d'une zone (6) sur laquelle se déplace l'au moins un appareil de compactage de sol (19) ;
générer un chemin de déplacement actualisé (3) à parcourir pour couvrir la zone d'ordre de travail (2) à compacter par l'au moins un appareil de compactage de sol (19) au moins une fois en utilisant
l'actionnement de compacteur pour le compactage de sol (S) et
une fonction de mesure pour mesurer le niveau de compactage du sol pendant le compactage ;
conduire le compacteur de sol (10) suivant le chemin de déplacement actualisé (3) ;
dans lequel le procédé comprend en outre :
dans le modèle tel que construit d'un niveau de compactage (50), sauvegarder au moins un niveau de compactage du sol tel que construit spécifique à un emplacement ;
mettre à jour le modèle tel que construit d'un niveau de compactage (50) de manière sensiblement continue, au moins au niveau de compactage du sol tel que construit spécifique à un emplacement ; et
mettre à jour le chemin de déplacement actualisé (3) en fonction du modèle tel que construit d'un niveau de compactage (50) mis à jour.

2. Procédé selon la revendication 1, dans lequel le chemin de déplacement actualisé (3) couvre la zone d'ordre de travail (2) de même que le nombre de fois où le niveau de compactage du sol tel que construit spécifique à un emplacement dépassant un niveau seuil de compactage du sol couvre la zone d'ordre de travail (2).

3. Procédé selon la revendication 2, dans lequel le modèle tel que construit d'un niveau de compactage (50) comprend en outre : des limites (2a, 2b, 2c, 2d) déterminées de la zone d'ordre de travail (2) à compacter et/ou des limites des zones dépassant le niveau seuil de compactage du sol.

4. Procédé selon l'une des revendications 1 à 3, dans lequel la génération du chemin de déplacement actualisé (3) se fait en plus de la zone d'ordre de travail (2) à compacter, en se basant au moins sur :
des propriétés physiques pour commander le compacteur de sol (10) ; et
des propriétés physiques de l'au moins un appareil de compactage de sol (19) du compacteur de sol (10).

5. Procédé selon l'une des revendications 1 à 4, dans lequel on met à jour le niveau de compactage du sol tel que construit spécifique à un emplacement sauvegardé dans le modèle tel que construit d'un niveau de compactage (50) à un niveau ultérieur d'un niveau de compactage du sol tel que construit spécifique à un emplacement par l'au moins un parmi : un remplacement ou une réécriture.

6. Procédé selon l'une des revendications 2 à 5, dans lequel on détermine le niveau seuil de compactage du sol qui est dépassé si un changement de pourcentage entre deux niveaux ultérieurs d'un niveau mesuré de compactage du sol spécifique à un emplacement est inférieur à un pourcentage seuil déterminé.

7. Procédé selon l'une des revendications 2 à 6, dans lequel le modèle tel que construit d'un niveau de compactage (50) comprend en outre au moins un parmi un niveau de hauteur tel que construit spécifique à un emplacement ou un nombre de niveaux spécifiques à un emplacement de niveaux mesurés de compactage du sol tels que construits spécifiques à un emplacement.

8. Procédé selon l'une des revendications 1 à 7, dans lequel les limites (2a, 2b, 2c, 2d) de la zone d'ordre de travail (2) à compacter sont déterminées par au moins une parmi :
la conduite manuelle et la sélection d'au moins un parmi : le bord le plus à droite ou le plus à gauche de la zone (6) sur laquelle se déplace l'au moins un appareil de compactage de sol (19) ;
une définition par l'utilisateur via une interface utilisateur ;
une indication dans un modèle d'informations de terrassement ; ou
une combinaison des éléments précédents.

9. Procédé selon l'une des revendications 1 à 8, dans lequel le chemin de déplacement actualisé (3) contient des transitions où au moins un parmi : l'actionnement de compacteur ou la fonction de mesure est désactivée.

10. Procédé selon l'une des revendications 1 à 9, dans lequel le chemin de déplacement actualisé (3) comprend la conduite avec actionnement de compacteur et sans actionnement de compacteur.

11. Procédé selon l'une des revendications 1 à 10, dans lequel le modèle tel que construit d'un niveau de compactage (50) peut être superposé à au moins un parmi : un modèle d'informations de terrassement ou une carte.

12. Procédé selon l'une des revendications 1 à 11, dans lequel le compacteur de sol (10) est conduit d'au moins l'une des manières suivantes : manuellement, semi-automatiquement ou automatiquement.

13. Compacteur de sol (10), le compacteur de sol (10) comprenant :
au moins un appareil de compactage de sol (19) doté d'un dispositif de mesure (20) pour le compactage de sol (S) et la mesure du niveau de compactage du sol pendant le compactage ;
un dispositif de navigation pour fournir des données d'emplacement et de direction de déplacement de l'au moins un appareil de compactage de sol (19) ;
un dispositif de conduite (14) pour conduire le compacteur de sol (10) ; et
un système de commande (40), dans lequel le système de commande (40) comprend au moins un processeur (41) et au moins une mémoire (42) comportant un code de programme informatique, l'au moins une mémoire (42) et le code de programme informatique étant configurés pour, avec l'au moins un processeur (41), amener le système de commande (40) à au moins :
générer un modèle tel que construit d'un niveau de compactage (50) couvrant au moins une zone d'ordre de travail (2) à compacter ;
recevoir des limites (2a, 2b, 2c, 2d) de la zone d'ordre de travail (2) à compacter ;
générer un chemin de déplacement actualisé (3) à parcourir pour couvrir la zone d'ordre de travail (2) à compacter au moins une fois ; et
dans le modèle tel que construit d'un niveau de compactage (50), sauvegarder au moins un niveau de compactage du sol tel que construit spécifique à un emplacement ;
mettre à jour le modèle tel que construit d'un niveau de compactage (50) de manière sensiblement continue, au moins au niveau de compactage du sol tel que construit spécifique à un emplacement ; et
mettre à jour le chemin de déplacement actualisé (3) en fonction du modèle tel que construit d'un niveau de compactage (50) mis à jour.

14. Compacteur de sol selon la revendication 13, dans lequel le système de commande (40) est en outre configuré pour :
recevoir un modèle d'informations de terrassement couvrant au moins la zone d'ordre de travail (2) indiquant la zone (6) à compacter.

15. Compacteur de sol selon la revendication 13 ou 14, dans lequel le compacteur de sol (10) est conduit d'au moins l'une des manières suivantes : manuellement, semi-automatiquement ou automatiquement.
